# EUROPEAN PATENT APPLICATION

(11) **EP 3 378 536 A1**
(43) Date of publication of application: **26.09.2018**
(21) Application number: 18158724.7
(22) Date of filing: 28.02.2013
(51) Int. Cl.: A61P 27/00, A61K 38/05, A23L 33/18, A61K 9/00, A61K 9/48, A61K 9/20, C07K 5/062

(54) **METHODS AND COMPOSITIONS FOR ENHANCEMENT OF VISION PERFORMANCE**

(30) Priority: 28.02.2012 US 201213407578
(62) Divisional of application: 13754020.9
(71) Applicant: Kyowa Hakko Bio Co., Ltd., Chiyoda-ku Tokyo 100-8185 (JP)
(72) Inventor: IKEDA, Takeshi, Tsukuba-shi, Ibaraki 305-8552 (JP); NEBASHI, Mami, Tsukuba-shi, Ibaraki 305-8552 (JP); MORISHITA, Koji, Tokyo, Ibaraki 100-8185 (JP); NAKAGIRI, Ryusuke, Aliso Viejo, CA California 92656 (US)
(74) Representative: Vossius & Partner Patentanwälte Rechtsanwälte mbB

(57) **Abstract**

A vision performance enhancing composition and method to enhance vision performance using alanylglutamine or a salt of alanylglutamine as an active ingredient.

## Description

### TECHNICAL FIELD OF THE INVENTION

The present invention relates to compositions containing alanylglutamine or a salt thereof as an active ingredient for the enhancement of vision performance.

### BACKGROUND OF THE INVENTION

The ability to see objects and respond to visual stimuli declines during periods of moderate to severe physical exertion. In particular, the ability to see and respond to stimuli presented in the periphery of the visual field declines compared to the ability to respond to visual stimuli presented in the central portion of the visual field (Ando et al., Int J Sports Med, Dec 2008; 29(12): 994-8. Epub 2008 Jul 3.).

Alanylglutamine is a dipeptide containing two amino acids, alanine and glutamine, and is immediately degraded into alanine and glutamine in the body (refer to "Clinical Science", 1988, Vol. 75, No. 5, p. 463-8). The action of glutamine is known to have many effects on physiological functions, such as the regulation of skeletal muscle protein metabolism, repair of small intestine mucosa, and improvement of immunofunction, and it has been reported that the effects of alanine on physiological functions include an action to suppress blood sugar levels in diabetes patients (refer to "L-Alanyl-L-Glutamine", Kyowa Hakko Co., Ltd., 2006, p. 1).

Alanylglutamine is superior in heat stability and solubility in aqueous solutions compared to glutamine, which has low-solubility and poor stability (refer to "L-Alanyl-L-Glutamine", Kyowa Hakko Co., Ltd., 2006, p. 3), and is used in parenteral nutritional agents as a glutamine supply source.

Nonetheless, alanylglutamine is not known to have an action to enhance vision performance.

### SUMMARY OF THE INVENTION

There is a demand for pharmaceutical products and nutritional foods, etc., that improve symptoms and create fulfilling lives for people having subjective symptoms of decreased vision performance attributed to exercise, including decreased peripheral vision and decreased visual reaction time. Specifically, an object of the present invention is to offer a composition which enhances vision performance.

One aspect of the present invention is a vision performance enhancing composition containing alanylglutamine or a salt thereof as an active ingredient.

Another aspect of the present invention is a method of enhancing vision performance by administering an effective amount of alanylglutamine or a salt thereof to a subject in need.

Yet another aspect of the present invention is a use of alanylglutamine or a salt thereof for producing a vision performance enhancing composition.

### BRIEF DESCRIPTION OF THE DRAWINGS

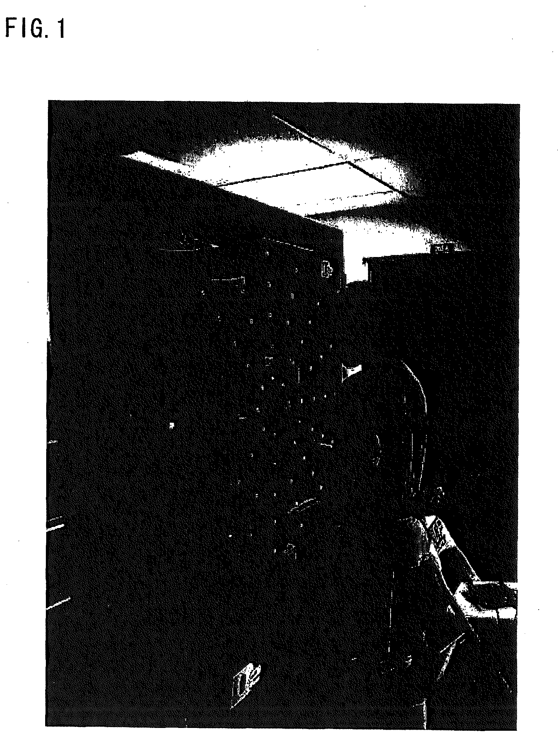

Figure 1 shows a subject participating in a trial.

Figure 2 is a graph depicting visual reaction time in subjects after administration of a vision performance enhancing composition of the invention.

Figure 3 is a graph depicting motor response time in subjects after administration of a vision performance enhancing composition of the invention.

### DETAILED DESCRIPTION OF THE INVENTION

In the composition of the present invention, alanine and glutamine are the amino acids that constitute alanylglutamine. Each may be L- or D-forms respectively, and the L-forms are preferred.

Salts of alanylglutamine include acid addition salts, metal salts, ammonium salts, organic amine addition salts, amino acid addition salts, and the like.

The acid addition salts include inorganic acid salts such as hydrochloride, hydrosulfate, nitrate and phosphate; and organic acid salts such as acetate, maleate, fumarate, citrate, malate, lactate, a-ketoglutarate, gluconate and caprylate.

The metal salts include alkali metal salts such as sodium salt and potassium salt; alkaline earth metal salts such as calcium salt; magnesium salt; aluminum salt; zinc salt, and the like.

Ammonium salts include salts of ammonium, tetramethylammonium, and the like.

Organic amine addition salts include salts of morpholine, piperidine, and the like.

Amino acid addition salts include salts of glycine, phenylalanine, lysine, aspartic acid, glutamic acid, and the like.

Alanylglutamine may be produced according to any method such as synthetic method, enzymatic method, or fermentation method.

Methods for producing alanylglutamine include, for example, those cited in Bulletin of the Chemical Society of Japan, 34, 739 (1961), 35, 1966 (1962), 37, 200 (1964), European Patent No. 311057, German Patent No. 3206784, Japanese Unexamined Patent Publication No. H6-234715, and WO2004/058960.

Commercial products (those manufactured by Kyowa Hakko, Co., Ltd., Kokusan Kagaku, Co., Ltd., and Bachem AG, etc.) may be used for alanylglutamine.

Vision performance can be improved by administering the compositions of the present invention to persons having decreased vision performance.

In the present invention, sports vision means the ability to see objects during sport activities, and includes peripheral vision during exercise and visual reaction time during exercise.

Alanylglutamine or a salt thereof may be administered as it is as the vision performance enhancing agent of the present invention, but preferably alanylglutamine is provided in any of a variety of pharmaceutical preparations.

These pharmaceutical preparations contain alanylglutamine or a salt thereof as an active ingredient, but may also contain any other therapeutic active ingredients. Further, these pharmaceutical preparations may be produced by any method well known in the technical field of pharmaceutics by mixing active ingredients with one or more pharmaceutically acceptable carriers.

It is desirable to use the pharmaceutical preparation through a dosing route that is the most effective for the therapy, and examples thereof include oral administration and parenteral administration such as intravenous administration, intraperitoneal administration, or subcutaneous administration; but oral administration is preferred.

The dosage form may be oral preparations, such as tablets, powders, granules, pills, suspensions, emulsions, infusions/decoctions, capsules, syrups, liquid preparations, elixirs, extracts, tinctures and fluid extracts, or parenteral preparations, such as injections, IV drip, creams and suppositories; but oral preparations are preferable.

When preparing oral preparations, excipients may be used such as fillers, binders, disintegrators, lubricants, dispersing agents, suspension agents, emulsifiers, diluents, buffers, antioxidant agents, microbial inhibitors, and the like.

Liquid preparations suitable to oral administration, for example, syrups, can be formulated by adding: water; a saccharide such as sucrose, sorbitol, or fructose; a glycol such as polyethylene glycol, or propylene glycol; an oil such as sesame oil, olive oil, or soybean oil; an antiseptic such as a p-hydroxybenzoate ester; a preservative such as a paraoxybenzoate derivative like methyl paraoxybenzoate or sodium benzoate; a flavor such as strawberry flavor or peppermint; or the like.

Further, for example, tablets, powders or granules, each of which is suitable for oral administration, can be formulated by adding: a saccharide such as lactose, sugar, glucose, sucrose, mannitol, or sorbitol; a starch such as that of potato, wheat, or corn; an inorganic substance such as calcium carbonate, calcium sulfate, sodium hydrogen carbonate, or sodium chloride; a filler such as crystalline cellulose or plant powder like licorice root powder, gentian powder, or the like; a disintegrator such as starch, agar, gelatin powder, crystalline cellulose, carmellose sodium, carmellose calcium, calcium carbonate, sodium hydrogen carbonate, or sodium alginate; a lubricant such as magnesium stearate, talc, hydrogenated plant oil, macrogol, or silicone oil; a binder such as polyvinyl alcohol, hydroxypropyl cellulose, methyl cellulose, ethyl cellulose, carmellose, gelatin, or starch paste; a surfactant such as a fatty acid ester; a plasticizer such as glycerol; or the like.

Additives generally used in foods or drinks may be added to preparations suitable for oral administration, including: sweeteners, colorants, preservatives, thickening stabilizers, antioxidant agents, coloring agents, bleaching agents, antifungal agents, gum bases, bitter agents, enzymes, waxes, sour agents, seasonings, emulsifiers, reinforcing agents, manufacturing agents, flavors, spice extracts, or the like.

The preparation suitable for oral administration may be used as a food or drink for improving vision performance such as a health food, a functional food, a nutritional supplement food, or a food for specific health use; and these may be in an unprocessed form or in such forms as a powdered food, a sheet-shaped food, a bottled food, a canned food, a retort food, a capsule food, a tablet food, a liquid food, or a drinkable preparation.

Suitable parenteral administration includes, for example, an injection that preferably contains a sterilized aqueous preparation containing alanylglutamine or a salt thereof, which is isotonic to the recipient's blood. In the case of an injection, for example, a solution for injection is prepared using a carrier containing a salt solution, a glucose solution, or a mixture of a salt solution and a glucose solution, or the like.

Further, also added to these parenteral preparations may be one or more auxiliary components selected from the diluents, antiseptics, flavors, fillers, disintegrators, lubricants, binders, surfactants and plasticizers described in the examples of the oral preparations, and the like.

In the compositions of the present invention, the concentration of alanylglutamine or a salt thereof is appropriately selected depending on the type of preparation, the effect expected by administration of the preparation, and the like, but, for example, the concentration in the case of an oral preparation is usually 0.1 to 100% by weight as alanylglutamine or a salt thereof, preferably 0.5 to 70% by weight, and particularly preferably 1 to 50% by weight.

The dose and the administration frequency of the compositions of the present invention may vary depending on the dosing form, the age and body weight of the patient, and the nature or the severity of the symptoms to be treated, but in general, it is administered once to several times a day usually in an amount of 5 mg to 10,000 mg, preferably 50 mg to 5,000 mg, more preferably 500 mg to 3,000 mg per day for an adult in terms of alanylglutamine or a salt thereof. The dosing period is not particularly limited, but is usually for 1 day to 1 year, preferably 2 weeks to 3 months.

### EXAMPLES

### Example 1: Manufacturing of a Tablet Containing Alanylglutamine

A mixture of 136.2 kg of alanylglutamine, 36.0 kg of microcrystalline cellulose, 6.6 kg of sucrose fatty acid ester, 1.2 kg of calcium phosphate, and 20.0 kg of [beta]-cyclodextrin are mixed using a conical blender (CB-1200 Blender, manufactured by Nihon Kansoki Co., Ltd.). The mixture obtained is compressed and molded to a tablet for of 250 mg with 8 mm of diameter under 10 kN of compression-molding pressure using a rotary compression molding machine (VIRGO524SS1AY, manufactured by Kikusui Seisakusho Co., Ltd.).

### Example 2: Manufacturing of an Enteric Capsule Containing Alanylglutamine

A mixture of 20 kg of the mixture produced in Example 1 and 0.2 kg of silicon dioxide are mixed and agitated. The mixture obtained is put into a capsule-filling machine to fill 20,000 tablets of gelatin Number 2 hard-capsules, and hard-capsules are obtained. The surfaces of the hard-capsules are coated with a zein solution using a High Coater HCT-48 (manufactured by Freund Corporation) to produce 20,000 enteric capsules.

### Example 3: Manufacturing of an Enteric Tablet Containing Alanylglutamine

The surfaces of the tablets produced in Example 1 are coated with a shellac solution using a High Coater HCT-48 (manufactured by Freund Corporation)' to produce enteric tablets.

### Example 4: Manufacturing of a Beverage Containing Alanylglutamine

An amount of 1.28 kg of alanylglutamine, 3 kg of erythritol (manufactured by Nikken Kagaku Co., Ltd.), 0.05 kg of citric acid, 3 g of artificial sweetener, and 0.06 kg of flavor are stirred and dissolved in 50 L of water at a temperature of 70°C. After the pH of the solution is adjusted to 3.3 with citric acid, the solution is sterilized using plate sterilization and filled into bottles. The bottles are sterilized using a pasteurizer, and thus a drink for enhancing vision performance is produced.

### Example 5: Efficacy of L-alanyl-L-glutamine (AG) Ingestion on Vision Performance

The purpose of this study was to examine the efficacy of L-alanyl-L-glutamine (AG) ingestion on sports vision, including reaction time. Ten women (21.2±1.6 years; height: 177.8±8.7 cm; body mass: 73.5±8.0 kg), all basketball players, volunteered for this study. The study required subjects to first participate in a 40 minute basketball game. During one trial, subjects consumed only water (W), while during the other two trials subjects consumed the AG supplement marketed as Sustamine mixed in water using either a low dose (1 g per 500 ml) (AG1) or high dose (2 g per 500 ml) (AG2) concentration. Other subjects where not allowed to consume water or AG (DHY).

Measurement of hand-eye reaction time was performed on the Dynavison D2 (Dynavision, Ontario Canada). Subjects were required to assume a comfortable athletic stance and stand at a distance from the board where they could easily reach all of the lights. The board height was adjusted so the LCD screen was located just below eye level. Participants were told to fixate their gaze on the LCD screen in the middle of the board and to keep their focus there for the entirety of the experiment. During the assessment each subject pressed a light with their dominant side index finger on the board. When a second light flashed (on the same line of the initial light, but on the non-dominant side of her body), the subject removed her finger and pressed the new visual stimulus. The time necessary to recognize the new stimulus (new light lit until finger lifted from initial light) was recorded as visual reaction time, and the time it took for the subject to move and press the newly lit light was recorded as the motor reaction time. The total time for both visual reaction and motor reaction was calculated as the physical reaction time. A total of eight attempts were performed. The average time for all eight attempts was recorded.

### Results

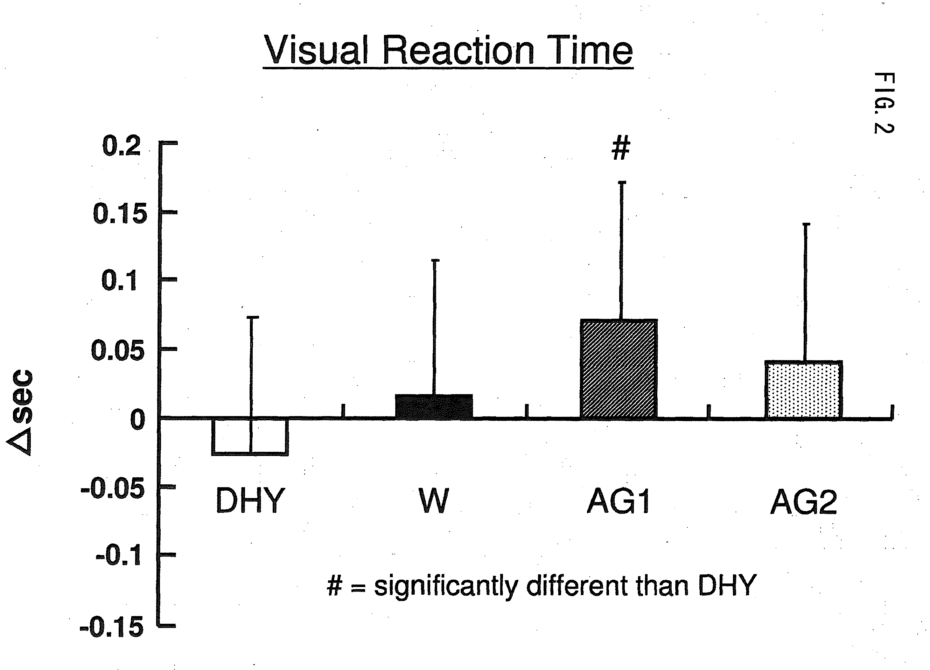

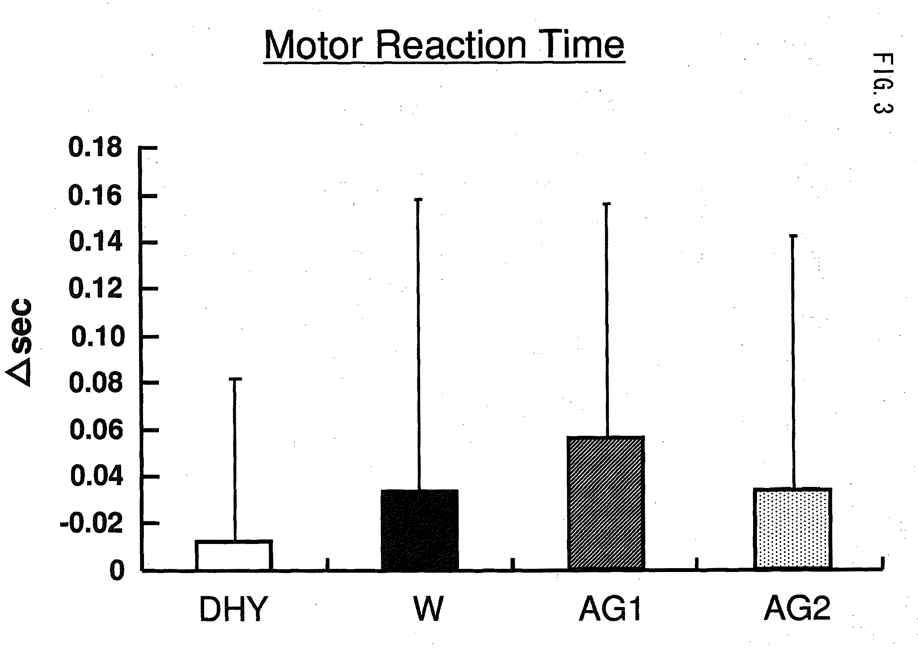

Visual reaction time (Figure 2) was significantly better following AG1 (p=0.014) compared to DHY (No water and no AG consumed during trial), and a trend toward a similar response (p=0.018) was noted between AG2 and DHY. However no significant differences were noted in the motor response (see Figure 3). The change in the physical reaction time (combined visual and motor differences) was significantly greater for AG1 compared to DHY (p=0.032).

Ingestion of AG1 also enhanced visual reaction time. The ability to enhance visual reaction time with AG1 does appear to have important implication for athletic performance.

According to the present invention, a sports vision improving agent containing alanylglutamine or a salt thereof as an active ingredient can be provided.

This application is based on US patent application No. 13/407,578, the contents of which are incorporated in full herein.

The invention further comprises the following items:
1. A vision performance enhancing composition comprising alanylglutamine or a salt thereof as an active ingredient.
2. A method of improving vision performance, comprising the step of administering an effective amount of alanylglutamine or a salt thereof to a subject in need.
3. A use of alanylglutamine or a salt thereof for producing a composition for enhancement of vision performance.
4. Alanylglutamine or a salt thereof for enhancement of vision performance.
5. The method of item 2, wherein the alanylglutamine or salt thereof is administered in an amount of 5 mg or more and 10,000 mg or less per day.
6. The method of item 2, wherein the alanylglutamine or salt thereof is administered in an amount of 50 mg or more and 5,000 mg or less per day.
7. The method of item 2, wherein the alanylglutamine or salt thereof is administered in an amount of 500 mg or more and 3,000 mg or less per day.

## Claims

1. A vision performance enhancing composition comprising alanylglutamine or a salt thereof as an active ingredient.

2. The vision performance enhancing composition of claim 1 for use in enhancing vision performance.

3. Alanylglutamine or a salt thereof for use in enhancement of vision performance.

4. The vision performance enhancing composition of claims 1 or 2 or the alanylglutamine or salt thereof of claim 3 for use in enhancement of vision performance, wherein the vision performance enhancing composition or the alanylglutamine or salt thereof is to be administered in an amount of 5 mg or more and 10,000 mg or less per day.

5. The vision performance enhancing composition or the alanylglutamine or salt thereof of claim 4 for use in enhancement of vision performance, wherein the vision performance enhancing composition or alanylglutamine or salt thereof is to be administered in an amount of 50 mg or more and 5,000 mg or less per day.

6. The vision performance enhancing composition or the alanylglutamine or salt thereof of claim 5 for use in enhancement of vision performance, wherein the vision performance enhancing composition or alanylglutamine or salt thereof is to be administered in an amount of 500 mg or more and 3,000 mg or less per day.
